**Europäisches Patentamt**

**European Patent Office**

**Office européen des brevets**

(19)

(11) Numéro de publication: **0 067 765**

**A1**

(12)

# DEMANDE DE BREVET EUROPEEN

(21) Numéro de dépôt: **82401031.8**

(22) Date de dépôt: **08.06.82**

(51) Int. Cl.³: **A 61 F 1/16**

(30) Priorité: **16.06.81 FR 8111842**

(43) Date de publication de la demande:
**22.12.82 Bulletin 82/51**

(84) Etats contractants désignés:
**AT BE CH DE GB IT LI LU NL SE**

(71) Demandeur: **Hanna, Khalil**
**5, rue Montmartre**
**F-75001 Paris(FR)**

(72) Inventeur: **Hanna, Khalil**
**5, rue Montmartre**
**F-75001 Paris(FR)**

(74) Mandataire: **Robert, Jean-Pierre et al,**
**CABINET BEAU DE LOMENIE 55, rue d'Amsterdam**
**F-75008 Paris(FR)**

(54) **Implant intraoculaire.**

(57) L'invention concerne un implant intra-oculaire constitué par une lentille 1 pourvue d'anses (3a, 4a, 3b, 3c, 4c, 4b) de fixation s'étendant à l'extérieur de la lentille de manière sensiblement radiale. Selon l'une des caractéristiques de l'invention, au moins l'une des anses antérieures (3a, 4a) est constituée par deux fils (3a, 4a) recourbés l'un vers l'autre et terminés par des pointes acérées (13a, 14a) se chevauchant en position de repos.

Fig-1

1

## Implant intra-oculaire.

La mise en place d'un cristallin artificiel est réalisée aujourd'hui de manière assez habituelle. Les solutions de fixation et de maintien en place des lentilles dans l'oeil, bien que maintenant satisfaisantes, restent toujours à perfectionner. On citera pour mémoire les lentilles qui sont maintenues dans l'oeil au moyen d'anses souples et légèrement élastiques qui prennent appui soit dans les angles des chambres antérieures ou postérieures de l'oeil selon que la lentille est placée dans la chambre correspondante, soit sur l'iris proprement dit qui se trouve légèrement pincé entre des anses antérieures et postérieures décalées angulairement. L'un des inconvénients majeurs de ce type de fixation réside dans les risques de luxation de l'implant lors des mouvements de l'iris et empêche toute dilatation de pupille à des fins d'observation de la rétine. En outre, la sollicitation mécanique qu'imposent ces anses aux tissus avec lesquels elles sont en contact provoque une gêne, une irritation, voire un traumatisme à ces tissus qui entraînent soit une intolérance, soit des complications qu'il convient soit de constamment traiter par voie de médicaments, soit d'opérer chirurgicalement. Une méthode d'implantation récente de ce type de cristallin artificiel consiste à suturer l'une ou deux des anses à l'iris afin d'éviter la luxation quand la pupille se dilate et ainsi de permettre un examen rétinien. Une telle suture demande de la part du chirurgien beaucoup d'habileté, d'expérience et de savoir-faire.

La présente invention entend fournir aux praticiens un implant simple à mettre en place et simple de conception de manière que sa manipulation tant du point de vue stérilisation par la chaleur que dans la pratique opératoire soit la plus aisée possible.

A cet effet, l'implant intra-oculaire selon l'invention est constitué par une lentille de verre et par au moins une paire d'anses antérieures pour sa fixation partant sensiblement radialement à partir de la lentille, chacune desdites anses étant constituée par un élément filiforme en boucle.

Selon l'une des caractéristiques de l'invention, ladite boucle est constituée par deux parties de fil élastique et recourbées l'une vers l'autre dans leur portion finale, de manière que leurs extrémités se chevauchent en position de repos pour réaliser une boucle fermée, les extrémités desdites portions finales étant taillées en pointes acérées.

En outre, chaque partie de fil élastique susdite d'une anse antérieure est constituée par une des parties terminales d'un fil continu traversant la lentille et formant une anse postérieure en forme de boucle, l'autre partie terminale du fil traversant également la lentille pour constituer une partie de fil élastique de l'autre anse antérieure.

La lentille peut comporter deux, trois ou quatre orifices de passage des fils susdits.

Enfin, lesdits fils sont pliés de manière à traverser la lentille perpendiculairement, les parties de fils ainsi orientées étant pourvues, devant ou derrière la lentille, d'entretoises d'épaisseur ou diamètre plus important que celui des fils ou orifices susdits.

L'invention sera mieux comprise au cours de la description donnée ci-après à titre d'exemple purement indicatif et non limitatif qui permettra d'en dégager les avantages et les caractéristiques secondaires.

Il sera fait référence aux dessins annexés dans lesquels :

- les figures 1 et 2 sont des vues respectivement de dessus et en coupe selon la ligne II-II de la figure 1 d'un mode de réalisation d'un implant selon l'invention ;

- la figure 3 illustre par un schéma l'implantation dans l'oeil du dispositif des figures 1 et 2 ; et

- les figures 4 et 5 illustrent deux variantes de réalisation de l'implant selon l'invention,

- les figures 6A, 6B, 7A et 7B illustrent deux autres variantes de réalisation de l'invention.

En se reportant tout d'abord aux deux premières figures, on voit un implant selon l'invention constitué par une lentille de verre 1 plan-convexe dans laquelle on a ménagé quatre orifices 2. Un premier élément filiforme 3 est passé dans deux des orifices 2 susdits pour former une demi-anse antérieure 3a, une anse postérieure 3b et une demi-anse antérieure 3c. De même, un second élément filiforme 4 forme une demi-anse antérieure 4a, une anse postérieure 4b et une demi-anse antérieure 4c. Cet élément 3 ou 4 est en un matériau élastique, par exemple en titane, de manière que les anses qu'il forme soient souples et élastiquement déformables. On notera que les portions finales 3a, 3c, 4a, 4c qui définissent les anses antérieures de fixation de l'implant sont recourbées l'une vers l'autre (3a vers 4a et 3c vers 4c), de manière qu'en position de repos leurs extrémités se recouvrent et constituent une boucle fermée. En outre, ces extrémités 13a, 13c et 14a, 14c sont taillées par affûtage en pointes acérées.

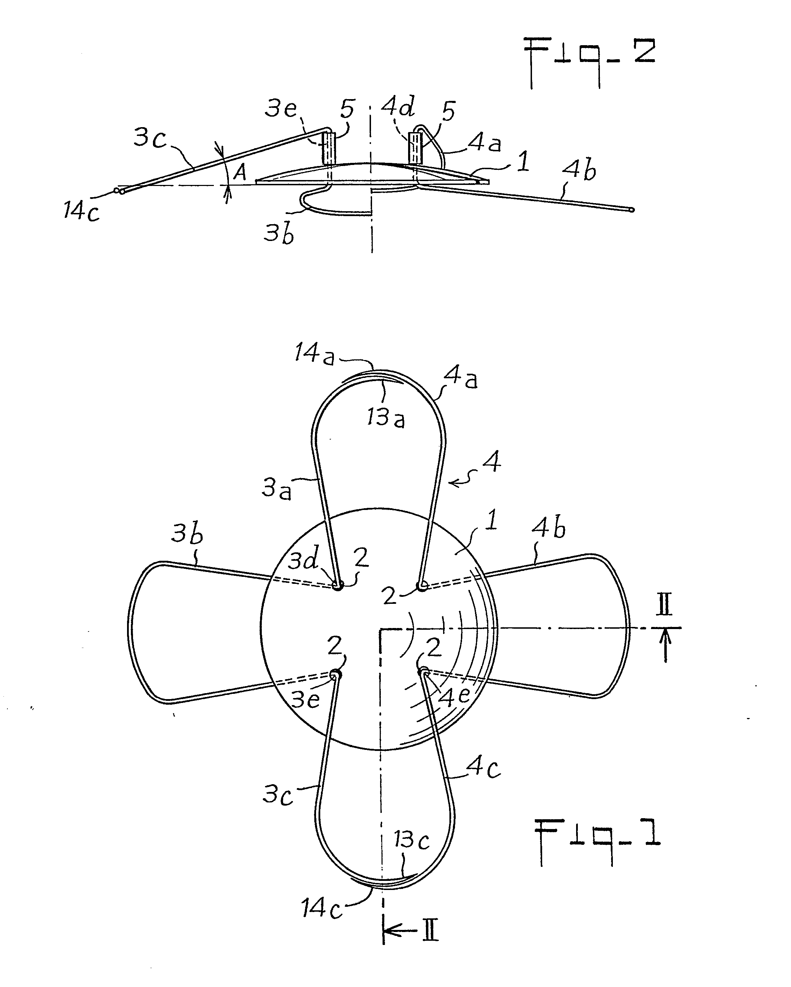

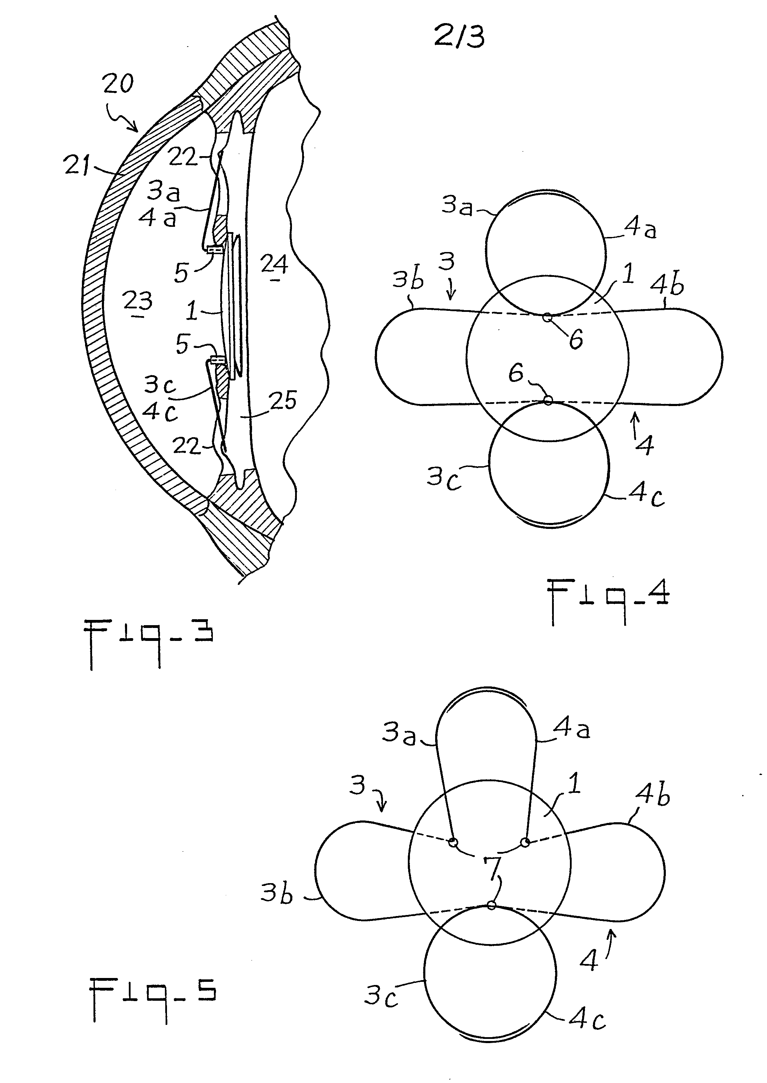

Par ailleurs, on remarquera que les parties antérieures, situées en avant de la lentille 1, de chacun des fils 3 et 4, sont reliées en une seule pièce, aux parties postérieures, à l'arrière de la lentille, au moyen de portions de fils, logées pour partie dans les orifices 2, perpendiculaires au plan de la lentille. Chacune de ces portions 3d, 3e pour le fil 3 et 4d, 4e pour le fil 4 est suffisamment longue pour pouvoir comporter à l'extérieur des orifices 2 une entretoise 5. Cette dernière, de diamètre supérieur à celui du fil ou des orifices 2, peut être située, comme représenté à la figure 2, en avant de la lentille ou, en revanche, derrière cette dernière. Enfin, on notera que les anses postérieures 3b, 4b sont sensiblement parallèles au plan de la lentille 1, alors que les anses antérieures 3a, 4a et 3c, 4c forment avec ce plan un angle A compris entre 10 et 20° et, de préférence, 15°.

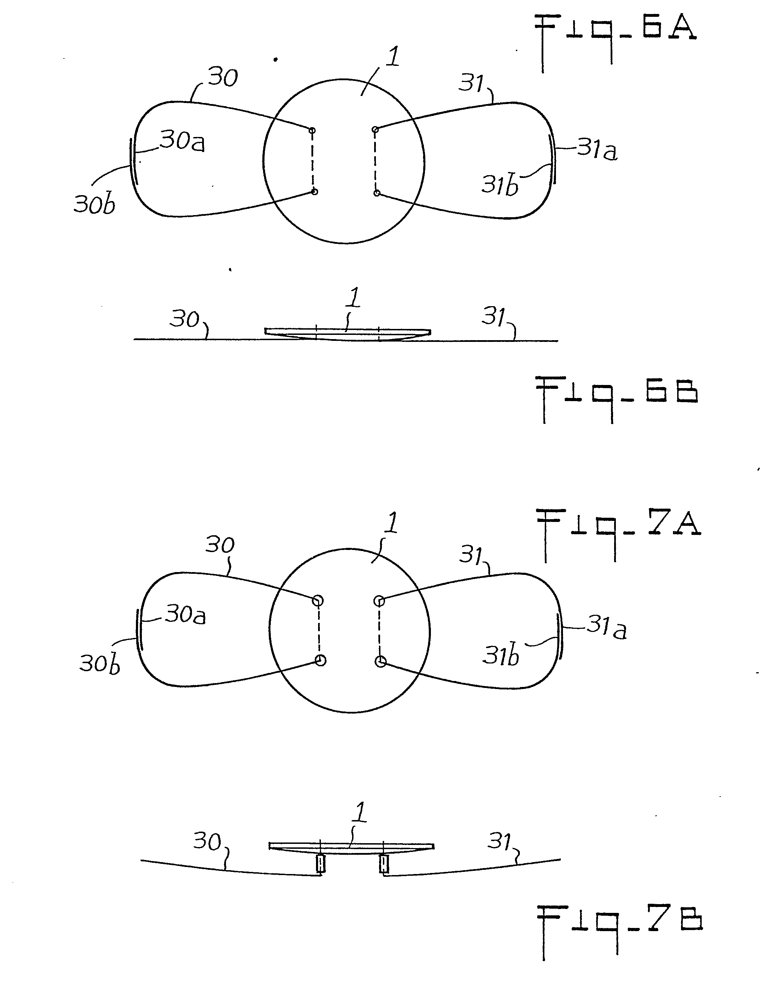

La figure 3 illustre par un schéma l'implant selon l'invention mis en place dans l'oeil. L'oeil 20 comprend notamment une cornée 21 définissant avec l'iris 22 une chambre antérieure 23. Derrière l'iris 22 mais devant le corps vitré 24, il existe une chambre 25 dite chambre postérieure. La mise en place du cristallin artificiel de l'invention nécessite les manipulations chirurgicales normales d'incision de la cornée et d'extraction du cristallin malade. On procède ensuite à l'insertion de l'implant en glissant latéralement les anses postérieures 3b, 4b derrière l'iris par un mouvement de va-et-vient latéral qui est relativement simple par rapport aux mouvements nécessités par les autres implants. Après la suture de l'incision cornéenne, la fixation de l'implant consiste uniquement à écarter les branches 3c, 4c, et 3a, 4a, des anses antérieures à l'aide de crochets très fins passés entre deux points de suture pour glisser entre elles une portion d'iris 22 qu'elles perforent sous l'effet de leur retour naturel élastique. La boucle qu'elles forment vient alors se refermer derrière l'iris comme le montre la figure 3. Dans cette position, aucune luxation de l'implant n'est plus possible même lors d'une dilatation pupillaire. On notera que les entretoises 5, qui peuvent être au contact du bord irien lorsque la pupille est petite, empêchent un effet de sciage de l'iris par les fils 3 et 4. Sur la figure 3, on a représenté une mise en place de l'implant dans la chambre postérieure 25 de l'oeil, les entretoises 5 étant alors placées en avant de la lentille 1. En revanche, si l'on place ces entretoises à l'arrière de la lentille, l'iris évolue derrière la lentille également et l'implant est placé dans la chambre antérieure. En tout état de cause, quelle que soit la chambre recevant l'implant, ce dernier est toujours placé dans l'aire pupillaire, ce qui est une disposition idéale pour une telle prothèse.

Les moyens essentiels de l'invention, à savoir au moins une anse antérieure divisée en deux parties rappelées élastiquement l'une vers l'autre en recouvrement et possédant des pointes effilées, peuvent être mis en oeuvre dans toute prothèse connue possédant des anses filiformes en métal ou en matière synthétique. Ils peuvent également être appliqués à des lentilles en matériau synthétique. Il est cependant préférable, pour des raisons de stérilisation, de stabilité dans le temps et de tolérance par l'organisme, d'adopter une lentille de verre et des anses métalliques telles que le titane ou autre matériau inerte.

L'implant selon l'invention permet de supprimer toute suture et notamment la suture inférieure qui est une opération délicate. Il permet également de réduire considérablement le temps de l'intervention.

On notera également un autre avantage de l'implant selon l'invention. Pour une lentille disposée dans la chambre postérieure, les anses antérieures constituent des points d'appui en cas d'effacement de la chambre antérieure. La surface de contact de l'endothélium avec la prothèse est de ce fait extrêmement réduite et un tel effacement ne conduit pas à l'endommagement de la plupart des cellules endothéliales comme cela est le cas avec des prothèses connues, à fixation irienne. .

Les figures 4 et 5 montrent des variantes de réalisation de l'implant selon l'invention. Dans la figure 4, la lentille 1 ne possède que deux orifices au travers desquels passent les deux fils 3 et 4 formant les anses de fixation. La lentille 1 de la figure 5 ne comporte que trois orifices 7, l'orifice inférieur étant traversé par les deux fils 3 et 4. On remarquera sur ces figures que, comme dans le cas précédent, on aura conservé deux anses postérieures disposées latéralement, afin de faciliter leur mise en place derrière l'iris.

Les entretoises 5 peuvent être soit en une seule pièce avec le fil 3 ou 4, soit rapportées sur ce dernier. Elles seront dans ce dernier cas constituées par un tronçon de tube en titane poli de hauteur égale sensiblement à un demi-millimètre qui sera déformé le long de sa génératrice tournée vers le centre de la lentille pour pincer la portion de fil 3d, 3e, 4d, 4e sur laquelle elle est placée. Le diamètre de ce tube pourra être de l'ordre de 0,2 mm à l'extérieur et 0,1 mm à l'intérieur.

On indiquera ci-après à titre d'exemple les caractéristiques dimensionnelles de l'implant selon l'invention dans toutes ses variantes :

lentille : - diamètre : 4 mm

- épaisseur au bord : 0,03 mm

- épaisseur au centre : 0,173 mm

- matériau : verre

- puissance : 19 dioptries

- volume : 1,273 $mm^3$

- poids dans l'air : 3,565 mg

- indice de réfraction : 1,6056

anses : - fil de titane de diamètre : 0,05 mm

diamètre total de l'implant : 8,5 à 9 mm

poids total dans l'eau : 2,497 mg

Les figures 6A, 6B et 7A, 7B montrent une lentille 1 qui ne comporte que deux anses 30 et 31. Ces anses sont sensiblement radiales et diamétralement opposées et comme dans les variantes précédentes, sont constituées par des fils, d'alliage de titane recourbés et terminés par des pointes acérées 30a, 30b, 31a, 31b se chevauchant. Dans le cas des figures 6A et 6B, les anses sont sensiblement dans le plan de la lentille. Dans le cas des figures 7A et 7B, on retrouve des entretoises tubulaires 5 comme dans les figures précédentes.

Les deux variantes ci-dessus présentent l'avantage de pouvoir être implantées de manière secondaire c'est-à-dire sur un oeil déjà opéré de la cataracte (surtout si cette dernière est unilatérale) et qui n'a pas supporté une lentille cornéenne. L'oeil est alors réopéré pour mettre en place un cristallin artificiel afin de rétablir une vision binoculaire.

Du fait de la géométrie de l'implant selon cette variante, l'incision cornéenne ou limbique nécessaire à sa mise en place est de petite dimension (inférieure à cinq millimètres). Les extrémités des anses sont, après fixation, situées en arrière de l'iris et ne touchent donc ni la cornée, ni l'angle de la chambre antérieure. Les entretoises tubulaires permettent de localiser la lentille dans le plan rétro-irien donc loin de la cornée et d'obtenir un centrage très précis de la lentille du fait de la pupille.

La version des figures 6A et 6B sans entretoises est très simple à introduire car la lentille et les anses sont dans le même plan. La plus grande dimension de l'implant peut être de 9 à 12 millimètres, ce qui est possible du fait de la situation postérieure à l'iris des pointes des anses et ce qui permet une dilatation complète de la pupille sans déformation.

Il faut noter par ailleurs que l'on peut améliorer d'une part l'état de surface des anses et empêcher l'oxydation du verre de la lentille, et d'autre part, l'inertie chimique et donc la tolérance et la stabilité dans le temps de l'implant selon l'invention en procédant à un dépôt d'une fine pellicule de quartz, sur les éléments le composant. Ce dépôt peut intervenir avant ou après leur assemblage.

7

L'invention trouve une application intéressante dans le domaine de la chirurgie de l'oeil.

Elle n'est pas limitée à la description qui vient d'en être donnée, mais couvre au contraire toutes les variantes qui pourraient lui être apportées sans sortir de son cadre ni de son esprit.

8

REVENDICATIONS
----------------------------

1.    Implant intra-oculaire formant cristallin artificiel constitué par une lentille (1) de verre et par au moins une paire d'anses (3a, 4a, 3c, 4c) antérieures pour sa fixation partant sensiblement radialement à partir de la lentille (1), chacune desdites anses étant constituée par un élément filiforme (3, 4) en boucle, caractérisé en ce que ladite boucle (3a, 4a) est constituée par deux parties (3a, 4a) de fil élastique et recourbées l'une vers l'autre dans leur portion finale, de manière que leurs extrémités (13a, 14a) se chevauchent en position de repos pour réaliser une boucle fermée, les extrémités desdites portions finales étant taillées en pointes acérées.

2.    Implant intra-oculaire selon la revendication 1, caractérisé en ce que chacune des boucles susdites forme un angle avec le plan de la lentille d'environ 10 à 20° qu'elles coupent à l'extérieur de cette dernière.

3.    Implant selon la revendication 1 ou la revendication 2, caractérisé en ce que chaque partie (3a) de fil élastique susdite d'une anse antérieure est constituée par une des parties terminales (3a) d'un fil (3) continu traversant la lentille et formant une anse postérieure (3b) en forme de boucle, l'autre partie terminale (3c) du fil traversant également la lentille pour constituer une partie de fil élastique de l'autre anse antérieure.

4.    Implant selon la revendication 3, caractérisé en ce que la lentille (1) comporte quatre orifices (2) de passage de chacun des deux fils susdits (3, 4).

5.    Implant selon la revendication 3, caractérisé en ce que la lentille comporte trois orifices (7) de passage de chacun des deux fils susdits (3, 4).

6.    Implant selon la revendication 3, caractérisé en ce que la lentille comporte deux orifices (6) de passage de chacun des deux fils susdits (3, 4).

7.    Implant selon l'une quelconque des revendications 4 à 6, caractérisé en ce que les fils susdits sont pliés de manière à présenter, au niveau des orifices, des tronçons sensiblement perpendiculaires (3d, 3e, 4d, 4e) à ladite lentille 1.

8.         Implant selon la revendication 7, caractérisé en ce que les tronçons susdits comportent des entretoises (5) d'épaisseur plus importante que le diamètre des fils ou orifices.

9.         Implant selon la revendication 8, caractérisé en ce que les entretoises (5) susdites sont situées en avant de la lentille.

10.         Implant selon la revendication 8, caractérisé en ce que les entretoises (5) susdites sont situées en arrière de la lentille.

11.         Implant selon la revendication 1, caractérisé en ce qu'il comporte uniquement deux anses antérieures (30, 31) diamétralement opposées, chacune d'elles étant formée par un fil aux extrémités (30a, 30b) en pointes acérées et se chevauchant au repos.

Fig. 2

Fig. 1

0067765

2/3

Fig-3

Fig-4

Fig-5

Fig. 6A

Fig. 6B

Fig. 7A

Fig. 7B

0067765

Office européen
des brevets

**RAPPORT DE RECHERCHE EUROPEENNE**

Numéro de la demande

EP 82 40 1031

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| A | FR-A-2 359 599  (REVLON) | 1 | A 61 F    1/16 |
| | --- | | |
| A | FR-A-2 260 322  (OTTER) | | |
| | ----- | | |

DOMAINES TECHNIQUES
RECHERCHES (Int. Cl. ³)

A 61 F

*Le présent rapport de recherche a été établi pour toutes les revendications*

| Lieu de la recherche | Date d'achèvement de la recherche | Examinateur |
|---|---|---|
| LA HAYE | 20-09-1982 | STEENBAKKER J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

 

& : membre de la même famille, document correspondant

OEB Form 1503. 03.82